# EUROPEAN PATENT APPLICATION

(11) **EP 2 984 985 A1**
(43) Date of publication of application: **17.02.2016**
(21) Application number: 15180462.2
(22) Date of filing: 11.08.2015
(51) Int. Cl.: A61B 5/00, A61B 5/06

(54) **PUNCTURE NEEDLE FOR PHOTOACOUSTIC IMAGER**

(30) Priority: 11.08.2014 JP 2014163903
(71) Applicant: PreXion Corporation, Chiyoda-ku Tokyo 101-0041 (JP)
(72) Inventor: NAKATSUKA, Hitoshi, Chiyoda-ku, Tokyo 101-0041 (JP)
(74) Representative: Grünecker Patent- und Rechtsanwälte PartG mbB

(57) **Abstract**

This puncture needle for a photoacoustic imager is a puncture needle (50, 150) for a photoacoustic imager (100, 200) including a semiconductor light-emitting element light source portion (10), and includes a puncture needle body (51) and a cover layer (52), formed on a surface (51d) of the puncture needle body, made of a cover material containing a material higher in light absorptivity than the puncture needle body and a resin material larger in linear expansion coefficient than the puncture needle body.

## Description

### BACKGROUND OF THE INVENTION

### Field of the Invention

The present invention relates to a puncture needle for a photoacoustic imager.

### Description of the Background Art

A photoacoustic imager including a puncture needle body is known in general, as disclosed in Japanese Patent Laying-Open No. 2013-27513.

Japanese Patent Laying-Open No. 2013-27513 discloses a photoacoustic imager including a laser beam source and a puncture needle body. This photoacoustic imager is configured to detect an acoustic wave signal (an acoustic wave) generated by the puncture needle body by applying a laser beam thereto from the laser beam source, so that the position of the puncture needle body in a specimen can be confirmed on a photoacoustic image based on the acoustic wave signal. In this photoacoustic imager, portions coated with a light scattering material and a light absorbing material respectively are provided on a surface of the puncture needle body. The photoacoustic imager is configured to lower the signal strength of the acoustic wave signal generated by the puncture needle body by adjusting the area of the portion coated with the light scattering material. Thus, the photoacoustic imager suppresses occurrence of an image artifact (turbulence of the image) resulting from the acoustic wave signal having high signal strength received from the puncture needle body. Consequently, the position of the puncture needle body can be confirmed on the photoacoustic image. In this photoacoustic imager, a general solid laser beam source is conceivably employed as a light source therefor.

When a light source employing a semiconductor light-emitting element such as an LED element is applied to the photoacoustic imager disclosed in Japanese Patent Laying-Open No. 2013-27513, however, the output of light is reduced as compared with the case of employing a solid laser beam source. In this case, the signal strength of the acoustic wave signal generated by the puncture needle body due to light applied from the semiconductor light-emitting element light source is lowered. While the puncture needle body described in the aforementioned Japanese Patent Laying-Open No. 2013-27513 is provided with the portion coated with the light absorbing material, the signal strength of the generated acoustic wave signal is positively reduced with the portion coated with the light scattering material. When a light source employing a semiconductor light-emitting element such as an LED element is employed, therefore, it is disadvantageously impossible to obtain an acoustic wave signal having signal strength sufficient for confirming the position of the puncture needle body on the photoacoustic image.

### SUMMARY OF THE INVENTION

The present invention has been proposed in order to solve the aforementioned problem, and an object of the present invention is to provide a puncture needle for a photoacoustic imager capable of more efficiently generating an acoustic wave so that the position of the puncture needle can be confirmed on a photoacoustic image also when employing a semiconductor light-emitting element such as an LED element (a light-emitting diode element) as a light source.

In order to attain the aforementioned object, a puncture needle for a photoacoustic imager according to an aspect of the present invention is a puncture needle for a photoacoustic imager including a semiconductor light-emitting element light source portion, and includes a puncture needle body and a cover layer, formed on a surface of the puncture needle body, made of a cover material containing a material higher in light absorptivity than the puncture needle body and a resin material larger in linear expansion coefficient than the puncture needle body.

In the puncture needle for a photoacoustic imager according to the aspect of the present invention, as hereinabove described, the cover layer made of a cover material containing a material higher in light absorptivity than the puncture needle body and a resin material larger in linear expansion coefficient than the puncture needle body is provided on the surface of the puncture needle body. Thus, light from the semiconductor light-emitting element light source portion can be applied to the cover layer, whereby the material having high light absorptivity can efficiently absorb the light received from the semiconductor light-emitting element light source portion, and the resin material having a large linear expansion coefficient can be efficiently thermally expanded with respect to heat resulting from the absorbed light. Consequently, the puncture needle can be efficiently thermally expanded with respect to the heat resulting from the absorbed light in addition to the absorption of the light due to the provision of the cover layer, whereby the same can more efficiently generate an acoustic wave. Therefore, the puncture needle can more efficiently generate an acoustic wave so that the position thereof can be confirmed on a photoacoustic image, also in the case of employing the semiconductor light-emitting element light source portion.

In the aforementioned puncture needle for a photoacoustic imager according to the aspect, the linear expansion coefficient of the resin material larger in linear expansion coefficient than the puncture needle body is preferably at least twice the linear expansion coefficient of the puncture needle body. According to this structure, the resin material larger in linear expansion coefficient than the puncture needle body can be reliably remarkably thermally expanded, whereby the puncture needle can further efficiently generate an acoustic wave.

In the aforementioned puncture needle for a photoacoustic imager according to the aspect, a light transmittance of the material higher in light absorptivity than the puncture needle body is preferably not more than 30 % at a measurement wavelength. According to this structure, the material having high light absorptivity with the light transmittance of not more than 30 % can reliably remarkably absorb the light received from the semiconductor light-emitting element light source portion, whereby the resin material having a large linear expansion coefficient can be reliably remarkably thermally expanded with respect to the heat resulting from the absorbed light. Consequently, the puncture needle can further efficiently generate an acoustic wave with respect to the absorbed light.

In the aforementioned puncture needle for a photoacoustic imager according to the aspect, the resin material larger in linear expansion coefficient than the puncture needle body preferably includes at least any one of an acrylic resin material, a fluororesin material and a silicone resin material. According to this structure, the resin material larger in linear expansion coefficient than the puncture needle body can be easily remarkably thermally expanded due to the employment of the resin material having a larger linear expansion coefficient as compared with a metallic material (SUS304, for example) generally employed for the puncture needle body.

In the aforementioned puncture needle for a photoacoustic imager according to the aspect, the cover layer is preferably provided in the form of a film having a planar surface on the surface of the puncture needle body. According to this structure, the specimen can be easily punctured with the puncture needle provided with the cover layer as compared with a case where the cover layer has no planar surface.

In the aforementioned structure in which the cover layer is provided in the form of a film having a planar surface, the cover layer preferably has a thickness of at least 5 µm and not more than 50 µm. According to this structure, the cover layer can reliably absorb light due to the thickness of at least 5 µm. Further, the cover layer can be easily formed and pain at the time of puncture can be relieved due to the thickness of not more than 50 µm.

In the aforementioned structure in which the cover layer is provided in the form of a film having a planar surface, the cover layer is preferably formed to cover the whole periphery of at least the needle tip of the puncture needle body on the surface of the puncture needle body. According to this structure, the position of the needle tip of the puncture needle body highly requiring imaging can be more reliably confirmed on the photoacoustic image.

In the aforementioned puncture needle for a photoacoustic imager according to the aspect, the material higher in light absorptivity than the puncture needle body is preferably a black-based material. According to this structure, the puncture needle can maintain high light absorptivity over a wide wavelength range independently of wavelength characteristics of the light from the semiconductor light-emitting element light source portion, due to the employment of the black-based material. Further, it is particularly effective in practice that the puncture needle can maintain high light absorptivity over a wide wavelength range in the photoacoustic imager generally varying a measurement wavelength with a detection object.

In this case, the black-based material preferably includes at least any one of carbon black, black-based iron oxide and black-based titanium oxynitride. According to this structure, the puncture needle can easily maintain high light absorptivity over a wide wavelength range due to the aforementioned material.

In the aforementioned structure in which the material higher in light absorptivity than the puncture needle body is a black-based material, the puncture needle body is preferably provided with a cut surface on the needle tip, the black-based material preferably includes black-based titanium oxynitride, and the cover layer preferably includes a marking portion formed by converting the cover material containing the black-based titanium oxynitride to white with heat so that the cut surface side of the needle tip of the puncture needle body is visually identifiable. According to this structure, the marking portion for visually identifying the cut surface can be easily formed through the characteristic of the black-based titanium oxynitride converted to white due to oxidation with heat in the structure in which the black-based material includes black-based titanium oxynitride. Consequently, a user of the puncture needle can identify the cut surface, whereby he/she can easily puncture the specimen with the puncture needle in a state setting the cut surface in response to the traveling direction of the puncture needle when puncturing the specimen with the same.

In the aforementioned structure in which the cover layer includes a marking portion, the marking portion is preferably provided in the form of a line extending along the extensional direction of the puncture needle body. According to this structure, the user can more easily identify the cut surface due to the easily identifiable marking portion in the form of a line.

In the aforementioned structure in which the cover layer includes a marking portion, the marking portion is preferably provided in the form of a graduation. According to this structure, the user can easily recognize the insertion depth of the puncture needle when he/she punctures the specimen with the puncture needle. Consequently, the user can easily puncture the specimen with the puncture needle up to a desired insertion depth.

In the aforementioned puncture needle for a photoacoustic imager according to the aspect, the semiconductor light-emitting element light source portion preferably includes a light-emitting diode element as a semiconductor light-emitting element. According to this structure, power consumption in the photoacoustic imager can be reduced by employing the light-emitting diode element requiring relatively small power consumption.

In the aforementioned puncture needle for a photoacoustic imager according to the aspect, the semiconductor light-emitting element light source portion preferably includes a semiconductor laser element as a semiconductor light-emitting element. According to this structure, a laser beam relatively higher in directivity as compared with the light-emitting diode element can be applied to the specimen, whereby most part of the light from the semiconductor laser element can be reliably applied to the specimen.

In the aforementioned puncture needle for a photoacoustic imager according to the aspect, the semiconductor light-emitting element light source portion preferably includes an organic light-emitting diode element as a semiconductor light-emitting element. According to this structure, the semiconductor light-emitting element light source portion can be easily miniaturized due to the employment of the organic light-emitting diode element easily reducible in thickness.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 shows the overall structure of a photoacoustic imager according to each of first and second embodiments of the present invention;
Fig. 2 is a schematic diagram showing a measuring state of a puncture needle of the photoacoustic imager according to the first embodiment of the present invention;
Fig. 3 is a schematic sectional view showing the puncture needle of the photoacoustic imager according to the first embodiment of the present invention;
Fig. 4 is a plan view showing the puncture needle of the photoacoustic imager according to the first embodiment of the present invention; and
Fig. 5 is a plan view showing a puncture needle of the photoacoustic imager according to the second embodiment of the present invention.

### DESCRIPTION OF THE PREFERRED EMBODIMENTS

Embodiments of the present invention are now described with reference to the drawings.

### (First Embodiment)

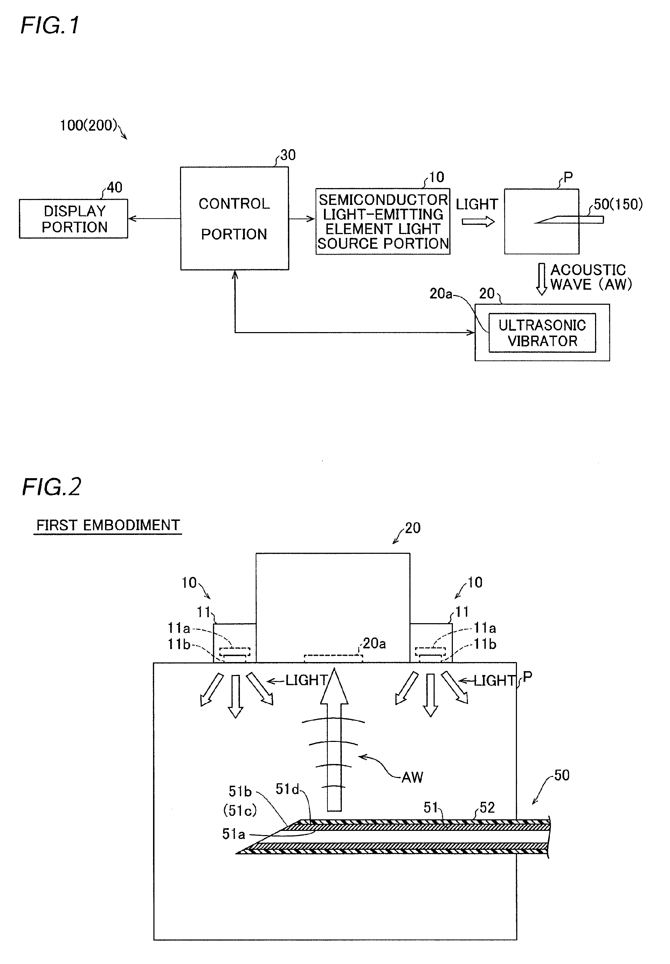

First, the structure of a photoacoustic imager 100 according to a first embodiment of the present invention is described with reference to Figs. 1 to 4.

The photoacoustic imager 100 according to the first embodiment of the present invention includes a semiconductor light-emitting element light source portion 10, a detection portion 20, a control portion 30, a display portion 40 and a puncture needle 50, as shown in Fig. 1. The puncture needle 50 is an example of the "puncture needle for a photoacoustic imager" in the present invention.

As shown in Figs. 1 and 2, the semiconductor light-emitting element light source portion 10 includes two semiconductor light-emitting element light sources 11, and is configured to apply pulsed light for measurement to a specimen P from each of the two semiconductor light-emitting element light sources 11. The two semiconductor light-emitting element light sources 11 are arranged in the vicinity of the detection portion 20 on different sides thereof, to hold the detection portion 20 therebetween. Therefore, the two semiconductor light-emitting element light sources 11 are configured to apply pulsed light toward the specimen P on positions different from each other.

Both of the two semiconductor light-emitting element light sources 11 have light source substrates 11a and semiconductor light-emitting elements 11b. For example, light-emitting diode elements, semiconductor laser elements or organic light-emitting diode elements can be employed as the semiconductor light-emitting elements 11b. A plurality of semiconductor light-emitting elements 11b are mounted in the form of arrays on the lower surfaces of the light source substrates 11a. The light source substrates 11a are provided with light source driving circuits, and configured to make the semiconductor light-emitting elements 11b emit pulsed light on the basis of control signals received from the control portion 30.

The semiconductor light-emitting elements 11b of both of the two semiconductor light-emitting element light sources 11 are configured to generate light (light having a peak wavelength in the range of about 700 nm to about 1000 nm, for example) of a measurement wavelength in the infrared region suitable for measurement of the specimen P such as a human body. The semiconductor light-emitting elements 11b of the two semiconductor light-emitting element light sources 11 may be configured to generate light of different measurement wavelengths or substantially identical measurement wavelengths. The measurement wavelengths may be properly decided in response to a detection object to be detected.

As shown in Figs. 1 and 2, the detection portion 20 has a plurality of arrayed ultrasonic vibrators 20a. The detection portion 20 is configured to detect an acoustic wave (an ultrasonic wave) through vibration of the ultrasonic vibrators 20a caused by the acoustic wave generated from the detection object, such as a puncture needle 50 in the specimen P, absorbing light applied from the semiconductor light-emitting element light source portion 10. Further, the detection portion 20 is configured to be capable of generating an ultrasonic wave by vibrating the ultrasonic vibrators 20a on the basis of a control signal received from the control portion 30. The detection portion 20 is configured to also detect the ultrasonic wave through vibration of the ultrasonic vibrators 20a caused by the ultrasonic wave reflected in the specimen P at this time. Further, the detection portion 20 is configured to output a detection signal corresponding to the detected acoustic or ultrasonic wave to the control portion 30.

Throughout the specification, an ultrasonic wave generated by the detection object, such as the puncture needle 50 in the specimen P, absorbing light is referred to as an "acoustic wave" and an ultrasonic wave generated by the ultrasonic vibrators 20a and reflected in the specimen P is referred to as an "ultrasonic wave" to be distinguished from each other, for the convenience of illustration.

The control portion 30 includes a CPU and a storage portion such as a ROM or a RAM, and is configured to perform imaging in the specimen P on the basis of a detection signal received from the detection portion 20. More specifically, the control portion 30 is configured to form a photoacoustic image on the basis of a detection signal resulting from an acoustic wave and to form an ultrasonic image on the basis of a detection signal resulting from an ultrasonic wave. Further, the control portion 30 is configured to be capable of imaging various information in the specimen P by integrating the photoacoustic and ultrasonic images.

As shown in Fig. 1, the display portion 40 is constituted of a general liquid crystal panel or the like, and configured to display the information in the specimen P imaged by the control portion 30 etc.

As shown in Figs. 1 and 2, the puncture needle 50 is a needle such as an injection needle employed for injecting a liquid medicine into the affected part of the specimen P or collecting an analyte (blood or the like) from the specimen P. Fig. 2 shows the puncture needle 50 in an exaggerated size with respect to the semiconductor light-emitting element light source portion 10 and the detection portion 20, for facilitating easy understanding of the present invention.

According to the first embodiment, the puncture needle 50 includes a puncture needle body 51 and a cover layer 52. The puncture needle body 51 is made of a metallic material (titanium, SUS304 or the like) excellent in biocompatibility. The cover layer 52, formed on the surface of the puncture needle body 51, is made of a cover material containing a black-based material for coloring the puncture needle body 51 black excellent in light absorptivity and a resin material generally larger in linear expansion coefficient as compared with the metallic material.

An easily colorable particulate black-based material can be employed as the black-based material. At least one of carbon black, black-based iron oxide and black-based titanium oxynitride can be employed as the particulate black-based material. The black-based titanium oxynitride excellent in biocompatibility and dispersibility is preferably employed as the black-based material.

The black-based material is higher in light absorptivity (smaller in light transmittance) than the puncture needle body 51 made of a metallic material having a light scattering property in general. The light transmittance of the black-based material is preferably not more than about 30 % at a measurement wavelength in the infrared region, and more preferably not more than about 25 %, in view of efficiently absorbing light. The light transmittance of the black-based material is more preferably not more than about 15 %, and further preferably not more than about 10 %.

At least any one of an acrylic resin material, a fluororesin material and a silicone resin material can be employed as the resin material.

The resin material has a linear expansion coefficient larger than that (about 1.73 × 10⁻⁶ K⁻¹) of SUS304 widely used as the metallic material for the puncture needle body 51 in general. The linear expansion coefficients of the acrylic resin material, the fluororesin material and the silicone resin material are at least about 5 × 10⁻⁶ K⁻¹ and not more than about 9 × 10⁻⁶ K⁻¹, at least about 6 × 10⁻⁶ K⁻¹ and not more than about 16 × 10⁻⁶ K⁻¹ and at least about 25 × 10⁻⁶ K⁻¹ and not more than about 30 × 10⁻⁶ K⁻¹ respectively, for example. In view of efficiently thermally expanding the resin material, therefore, the silicone resin material having the large linear expansion coefficient is preferably employed as the resin material.

The linear expansion coefficient of the resin material is preferably at least about twice, and more preferably at least about three times the linear expansion coefficient of the puncture needle body 51, in view of efficiently thermally expanding the resin material. The linear expansion coefficient of the resin material is more preferably at least about 5 times, and further preferably at least 10 times the linear expansion coefficient of the puncture needle body 51.

The resin material preferably has a refractive index close to the refractive index (about 1.3) of the biological tissue of the specimen P such as a human body, in view of suppressing reflection of light on the boundary surface between the cover layer 52 and the biological tissue of the specimen P such as a human body. The refractive indices of the acrylic resin material, the fluororesin material and the silicone resin material are about 1.5, about 1.35 and about 1.43 respectively, for example. In view of suppressing reflection of light on the boundary surface, therefore, the fluororesin material having the refractive index close to that of the biological tissue of the specimen P is preferably employed as the resin material. The fluororesin material is preferable also in view of excellent characteristics such as chemical resistance, nonviscousness, slidableness and non-wettability. PTFE, PFA, FEP, PCTFE, ETFE, ECTFE or PVDF can be employed as the fluororesin material.

In view of smoothly forming the cover layer 52 (to have a planar surface), the acrylic resin material capable of forming a smooth and uniform film by electrodeposition is preferably employed.

The weight ratio of the black-based material with respect to the weight of the entire cover material is preferably at least about 1 % and not more than about 5 %. The weight ratio of the resin material with respect to the weight of the entire cover material is preferably at least about 16 % and not more than about 30 %.

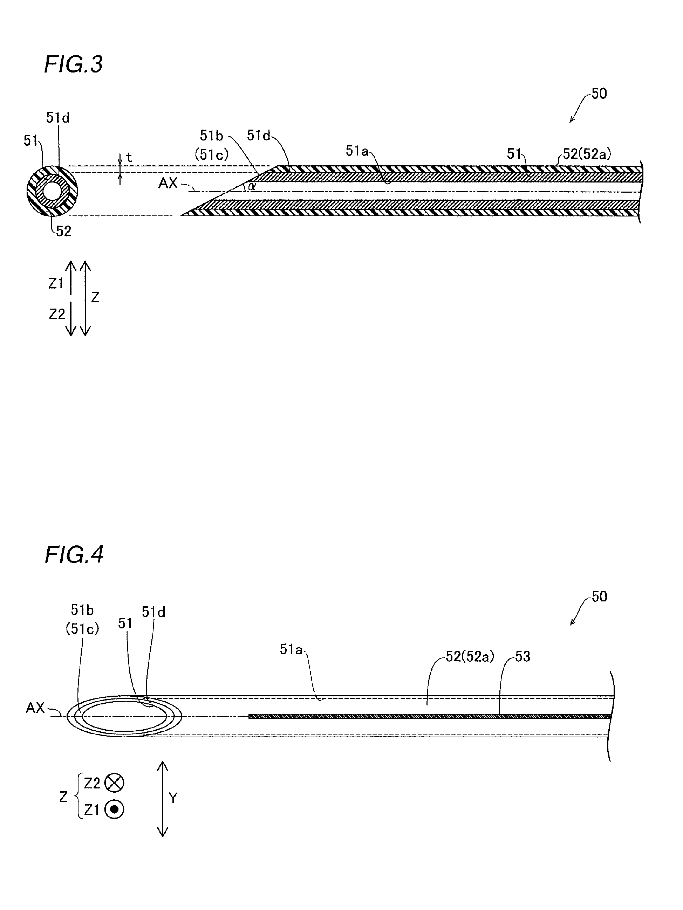

As shown in Figs. 2 and 3, the puncture needle body 51 has a pipelike needle tube 51a and a needle tip 51b formed on the forward end of the needle tube 51a. The needle tip 51b is provided with a cut surface 51c forming a sharp angle α with the central axis AX of the needle tube 51a.

The cover layer 52 covers an outer surface 51d of the needle tube 51a, and is provided in the form of a smooth film allowing easy puncture. Further, the cover layer 52 is formed up to at least a prescribed position (several cm from the needle tip 51b) inserted into the specimen P at the time of puncture from the needle tip 51b toward an end portion of the needle tube 51a opposite to the side provided with the needle tip 51b while covering the whole periphery of the outer surface 51d of the pipelike needle tube 51a. The cover layer 52 can be formed on the outer surface 51d of the needle tube 51a in the form of a film by a method such as coating. Figs. 2 to 4 show the needle tube 51a and the cover layer 52 in slightly exaggerated thicknesses with respect to the hole diameter of the puncture needle 50, for facilitating easy understanding of the present invention.

The thickness t of the cover layer 52 is preferably at least about 5 µm and more preferably at least about 10 µm, so that the cover layer 52 reliably absorbs light. Further, the thickness t of the cover layer 52 is preferably not more than about 50 µm and more preferably not more than about 30 µm, in view of easiness in production and in view of relieving pain at the time of puncture.

According to the first embodiment, the cover layer 52 is provided with a marking portion 53 (shown in a hatched manner) enabling visual identification of the cut surface 51c on a side (Z1 side) of the exposed outer surface 52a closer to the cut surface 51c of the puncture needle body 51, as shown in Fig. 4. The marking portion 53 is provided in the form of a line from a position separating from the needle tip 51b toward the extensional direction of the needle tube 51a at a substantially central position in the width direction (direction Y) of the needle tube 51a.

When a cover material containing black-based titanium oxynitride as the black-based material is employed, the marking portion 53 is formed by oxidizing the black-based titanium oxynitride with heat of a laser beam and converting the same to white titanium oxide. It is known that the black-based titanium oxynitride is oxidized with heat of at least 300°C to be converted to white titanium oxide.

In other words, the cover layer 52 is black as a whole due to the employment of the black-based material while the marking portion 53 is white, and hence the marking portion 53 can be formed in clear contrast with respect to the cover layer 52 according to the aforementioned method. Therefore, a user of the puncture needle 50 can easily visually recognize the marking portion 53, thereby easily identifying the cut surface 51c.

An operation of puncturing the specimen P with the puncture needle 50 and generation of an acoustic wave from the puncture needle 50 in the specimen P are now described with reference to Figs. 1 and 2.

First, the user (the person performing the puncturing operation) identifies the cut surface 51c by visually recognizing the marking portion 53 of the puncture needle 50. At the time of the puncture, the puncture needle 50 easily progresses upward in the specimen P when the cut surface 51c is directed downward, while the puncture needle 50 easily progresses downward in the specimen P when the cut surface 51c is directed upward (as shown in Fig. 2). Therefore, the upward/downward direction of the cut surface 51c at the time of the puncture depends on a position where the needle tip 51b of the puncture needle 50 is desired to reach.

After identifying the cut surface 51c through the marking portion 53 (see Fig. 4) and deciding the upward/downward direction of the cut surface 51c, the user punctures the specimen P with the puncture needle 50. In the state where the specimen P is punctured with the puncture needle 50, the semiconductor light-emitting element light source portion 10 applies pulsed light for measurement.

The cover layer 52 formed on the outer surface 51d of the puncture needle body 51 with a sufficient thickness absorbs the applied pulsed light. At this time, the black-based material having high light absorptivity (small light transmittance) efficiently absorbs the light. Then, the black-based material absorbing the light generates heat. The heat generated by the black-based material is transmitted to the resin material having a large linear expansion coefficient, to thermally expand the same. Consequently, the cover layer 52 can efficiently generate an acoustic wave AW, as compared with a case where only the puncture needle body 51 absorbs light. The cover layer 52 generates the acoustic wave AW not only by thermal expansion of the resin material resulting from the heat transmitted from the black-based material but also by thermal expansion of the black-based material resulting from the absorption of the light. Further, the cover layer 52 generates the acoustic wave AW also by thermal expansion of the resin material resulting from the absorption of the light.

Thereafter the acoustic wave AW propagating through the specimen P reaches the detection portion 20 to be detected by the detection portion 20, which in turn outputs a detection signal resulting from the acoustic wave AW to the control portion 30, as shown in Fig. 1. Then, the control portion 30 forms a photoacoustic image on the basis of the detection signal resulting from the acoustic wave AW, and the display portion 40 displays the formed photoacoustic image. Consequently, the user can confirm the position of the puncture needle 50 in the specimen P on the photoacoustic image.

According to the first embodiment, the following effects can be attained:
According to the first embodiment, as hereinabove described, the cover layer 52 made of the cover material containing the black-based material higher in light absorptivity than the puncture needle body 51 and the resin material larger in linear expansion coefficient than the puncture needle body 51 is provided on the surface (the outer surface 51d) of the puncture needle body 51. Thus, the light from the semiconductor light-emitting element light source portion 10 can be applied to the cover layer 52, whereby the black-based material having high light absorptivity can efficiently absorb the light from the semiconductor light-emitting element light source portion 10, and the resin material having a large linear expansion coefficient can be efficiently thermally expanded with respect to the heat resulting from the absorbed light. Consequently, thermal expansion with respect to the heat resulting from the absorbed light can be efficiently performed in addition to the absorption of the light due to the provision of the cover layer 52, whereby the acoustic wave AW can be more efficiently generated. Therefore, the puncture needle 50 can more efficiently generate the acoustic wave AW so that the user can confirm the position of the puncture needle 50 on the photoacoustic image, also in the case of employing the semiconductor light-emitting element light source portion 10.

According to the first embodiment, as hereinabove described, the linear expansion coefficient of the resin material larger in linear expansion coefficient than the puncture needle body 51 is at least about twice the linear expansion coefficient of the puncture needle body 51. Thus, the resin material larger in linear expansion coefficient than the puncture needle body 51 can be reliably remarkably thermally expanded, whereby the puncture needle 50 can further efficiently generate the acoustic wave AW.

According to the first embodiment, as hereinabove described, a light transmittance of the black-based material higher in light absorptivity than the puncture needle body 51 is not more than about 30 % at the measurement wavelength. Thus, the black-based material having high light absorptivity with the light transmittance of not more than about 30 % can reliably remarkably absorb the light received from the semiconductor light-emitting element light source portion 10, whereby the resin material having a large linear expansion coefficient can be reliably remarkably thermally expanded with respect to the heat resulting from the absorbed light. Consequently, the puncture needle 50 can further efficiently generate the acoustic wave AW with respect to the absorbed light.

According to the first embodiment, as hereinabove described, the resin material larger in linear expansion coefficient than the puncture needle body 51 includes at least any one of the acrylic resin material, the fluororesin material and the silicone resin material. Thus, the resin material larger in linear expansion coefficient than the puncture needle body 51 can be easily remarkably thermally expanded due to the employment of the resin material having a larger linear expansion coefficient as compared with a metallic material (SUS304, for example) generally employed for the puncture needle body 51.

According to the first embodiment, as hereinabove described, the cover layer 52 is provided in the form of a film having a planar surface on the surface of the puncture needle body 51. Thus, the specimen P can be easily punctured with the puncture needle 50 provided with the cover layer 52, as compared with a case where the cover layer 52 has no planar surface.

According to the first embodiment, as hereinabove described, the cover layer 52 has the thickness of at least 5 µm and not more than 50 µm. Thus, the cover layer 52 can reliably absorb the light due to the thickness of at least 5 µm. Further, the cover layer 52 can be easily formed and pain at the time of puncture can be relieved due to the thickness of not more than 50 µm.

According to the first embodiment, as hereinabove described, the cover layer 52 is formed to cover the whole periphery of at least the needle tip 51b of the puncture needle body 51 on the surface of the puncture needle body 51. Thus, the user can more reliably confirm the position of the needle tip 51b of the puncture needle body 51 highly requiring imaging on the photoacoustic image.

According to the first embodiment, as hereinabove described, the black-based material is higher in light absorptivity than the puncture needle body 51. Thus, the puncture needle 50 can maintain high light absorptivity over a wide wavelength range independently of wavelength characteristics of the light from the semiconductor light-emitting element light source portion 10, due to the employment of the black-based material. Further, it is particularly effective in practice that the puncture needle 50 can maintain high light absorptivity over a wide wavelength range as described above in the photoacoustic imager 100 generally varying the measurement wavelength with the detection object.

According to the first embodiment, as hereinabove described, the black-based material includes at least any one of carbon black, black-based iron oxide and black-based titanium oxynitride. Thus, the puncture needle 50 can easily maintain high light absorptivity over a wide wavelength range due to the aforementioned material.

According to the first embodiment, as hereinabove described, the cover layer 52 is provided with the marking portion 53 formed by converting the cover material containing the black-based titanium oxynitride to white with heat so that the user can visually identify the side of the cut surface 51c of the needle tip 51b of the puncture needle body 51. Thus, the marking portion 53 for facilitating easy visual identification of the cut surface 51c can be easily formed in the structure in which the black-based material includes the black-based titanium oxynitride through the characteristic of the black-based titanium oxynitride converted to white due to oxidation with heat. Consequently, the user can identify the cut surface 51c, whereby he/she can easily puncture the specimen P with the puncture needle 50 in a state setting the cut surface 51c in response to the traveling direction of the puncture needle 50 when puncturing the specimen P with the same.

According to the first embodiment, as hereinabove described, the semiconductor light-emitting elements 11b include at least light-emitting diode elements, semiconductor laser elements or organic light-emitting diode elements. When light-emitting diode elements are employed as the semiconductor light-emitting elements 11b, power consumption in the photoacoustic imager 100 can be reduced due to the employment of the light-emitting diode elements requiring relatively small power consumption. When semiconductor laser elements are employed as the semiconductor light-emitting elements 11b, laser beams having relatively high directivity can be applied to the specimen P, whereby most part of the light from the semiconductor laser elements can be reliably applied to the specimen P. When organic light-emitting diode elements are employed as the semiconductor light-emitting elements 11b, the semiconductor light-emitting element light source portion 10 provided therewith can be easily miniaturized due to the employment of the organic light-emitting diode elements easily reducible in thickness.

According to the first embodiment, as hereinabove described, the marking portion 53 is provided in the form of a line extending along the extensional direction of the puncture needle body 51. Thus, the user can more easily identify the cut surface 51c due to the formation of the easily identifiable linear marking portion 51.

### (Second Embodiment)

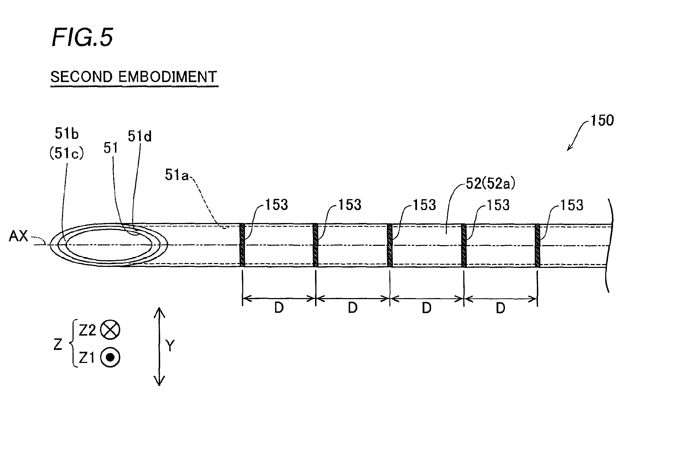

A photoacoustic imager 200 according to a second embodiment of the present invention is now described with reference to Figs. 1 and 5. According to the second embodiment, the photoacoustic imager 200 is provided with a plurality of (five) marking portions 153 in the form of graduations, dissimilarly to the structure of the photoacoustic imager 100 according to the aforementioned first embodiment having the marking portion 53 provided in the form of a line directed toward the extensional direction of the needle tube 51a. Structures identical to those of the aforementioned first embodiment are denoted by the same reference signs, and redundant description is omitted.

The photoacoustic imager 200 according to the second embodiment of the present invention includes a puncture needle 150, as shown in Fig. 1. The puncture needle 150 is substantially identical in structure to the puncture needle 50 according to the aforementioned first embodiment, except that the marking portions 153 are different from the marking portion 53 according to the first embodiment. In the puncture needle 150, a cover layer 52 is provided on an outer surface 51d of a puncture needle body 51, while the marking portions 153 (shown in a hatched manner) are provided on the cover layer 52. The puncture needle 150 is an example of the "puncture needle for a photoacoustic imager" in the present invention.

According to the second embodiment, the plurality of (five) marking portions 153 are provided on a side (Z1 side) of an exposed outer surface 52a closer to a cut surface 51c of the puncture needle body 51. The plurality of marking portions 153 are provided in the form of lines extending in the width direction (direction Y) of a needle tube 51a respectively, and formed on positions separating from each other by prescribed intervals D. In other words, the marking portions 153 function as graduations for measuring the insertion depth of the puncture needle 150 when a specimen P is punctured with the puncture needle 150, in addition to a function for facilitating easy visual identification of the cut surface 51c. The marking portions 153 functioning as graduations may alternatively be provided in a number other than five.

According to the second embodiment, the plurality of marking portions 153 are provided in the form of lines in a range longer than the half periphery of the outer surface 52a of the cover layer 52 and shorter than the whole periphery thereof respectively. In other words, the plurality of marking portions 153 are continuously formed on the side (Z1 side) of the cut surface 51c, and segmented on a side (Z2 side) opposite to the cut surface 51c. Consequently, the cut surface 51c is easily identifiable while the marking portions 153 are formed over a wide range, whereby the user can visually recognize the marking portions 153 in the form of graduations for measuring the insertion depth of the puncture needle 150 whether the cut surface 51c is directed upward or downward.

The remaining structures of the second embodiment are similar to those of the aforementioned first embodiment.

According to the second embodiment, the following effect can be attained:
According to the second embodiment, as hereinabove described, the marking portions 153 are provided in the form of graduations. Thus, the user can easily recognize the insertion depth of the puncture needle 150 when he/she punctures the specimen P with the puncture needle 150. Consequently, the user can easily puncture the specimen P with the puncture needle 150 up to a desired insertion depth.

The remaining effects of the second embodiment are similar to those of the aforementioned first embodiment.

Although the present invention has been described and illustrated in detail, it is clearly understood that the same is by way of illustration and example only and is not to be taken by way of limitation, the spirit and scope of the present invention being limited only by the terms of the appended claims.

For example, while the cover layer 52 covers the whole periphery of the outer surface 51d of the puncture needle body 51 in each of the aforementioned first and second embodiments, the present invention is not restricted to this. According to the present invention, the cover layer may not cover the whole periphery of the outer surface of the puncture needle body. According to the present invention, the cover layer may alternatively cover only part of the puncture needle body so far as the puncture needle is measurable. Further alternatively, the cover layer may cover the inner surface of the puncture needle body.

While the linear expansion coefficient of the resin material larger in linear expansion coefficient than the puncture needle body 51 is at least about twice the linear expansion coefficient of the puncture needle body 51 in each of the aforementioned first and second embodiments, the present invention is not restricted to this. According to the present invention, the linear expansion coefficient of the resin material may alternatively be smaller than about twice the linear expansion coefficient of the puncture needle body, so far as the linear expansion coefficient of the resin material is larger than that of the puncture needle body.

While the light transmittance of the black-based material higher in light absorptivity than the puncture needle body 51 is not more than about 30 % at the measurement wavelength in each of the aforementioned first and second embodiments, the present invention is not restricted to this. According to the present invention, the light transmittance of the black-based material may alternatively be larger than about 30 % at the measurement wavelength.

While the black-based material such as carbon black is employed as the material higher in light absorptivity than the puncture needle body 51 in each of the aforementioned first and second embodiments, the present invention is not restricted to this. According to the present invention, a material other than the black-based material may alternatively be employed, so far as the material has high light absorptivity (small light transmittance) at the measurement wavelength in the infrared region.

While the marking portion 53 (the marking portions 153) is (are) provided on the side of the cut surface 51c in each of the aforementioned first and second embodiments, the present invention is not restricted to this. According to the present invention, the marking portion(s) may not be provided on the side of the cut surface, so far as the side of the cut surface is visually identifiable. For example, the marking portion(s) may be provided only on the side opposite to the cut surface so that the user identifies the cut surface, or marking portions having different shapes may be provided on the cut surface side and the side opposite to the cut surface so that the user identifies the cut surface.

While the marking portion 53 is formed by converting the black-based titanium oxynitride to white titanium oxide with the heat of the laser beam when employing the cover material containing the black-based titanium oxynitride in the aforementioned first embodiment, the present invention is not restricted to this. According to the present invention, the marking portion may alternatively be formed by a method other than the above. For example, the marking portion may be formed by coating part of the cover layer with a paint, or by not providing the cover layer on part of the puncture needle body.

## Claims

1. A puncture needle (50, 150) for a photoacoustic imager (100, 200) comprising a semiconductor light-emitting element light source portion (10), comprising:
a puncture needle body (51); and
a cover layer (52), formed on a surface (51d) of the puncture needle body, made of a cover material containing a material higher in light absorptivity than the puncture needle body and a resin material larger in linear expansion coefficient than the puncture needle body.

2. The puncture needle for a photoacoustic imager according to claim 1, wherein
the linear expansion coefficient of the resin material larger in linear expansion coefficient than the puncture needle body is at least twice the linear expansion coefficient of the puncture needle body.

3. The puncture needle for a photoacoustic imager according to claim 1 or 2, wherein
a light transmittance of the material higher in light absorptivity than the puncture needle body is not more than 30 % at a measurement wavelength.

4. The puncture needle for a photoacoustic imager according to any of claims 1 to 3, wherein
the resin material larger in linear expansion coefficient than the puncture needle body includes at least any one of an acrylic resin material, a fluororesin material and a silicone resin material.

5. The puncture needle for a photoacoustic imager according to any of claims 1 to 4, wherein
the cover layer is provided in the form of a film having a planar surface on the surface of the puncture needle body.

6. The puncture needle for a photoacoustic imager according to claim 5, wherein
the cover layer has a thickness of at least 5 µm and not more than 50 µm.

7. The puncture needle for a photoacoustic imager according to claim 5 or 6, wherein
the cover layer is formed to cover the whole periphery of at least the needle tip (51b) of the puncture needle body on the surface of the puncture needle body.

8. The puncture needle for a photoacoustic imager according to any of claims 1 to 7, wherein
the material higher in light absorptivity than the puncture needle body is a black-based material.

9. The puncture needle for a photoacoustic imager according to claim 8, wherein
the black-based material includes at least any one of carbon black, black-based iron oxide and black-based titanium oxynitride.

10. The puncture needle for a photoacoustic imager according to claim 8, wherein
the puncture needle body is provided with a cut surface (51c) on the needle tip,
the black-based material includes black-based titanium oxynitride, and
the cover layer includes a marking portion (53, 153) formed by converting the cover material containing the black-based titanium oxynitride to white with heat so that the cut surface side of the needle tip of the puncture needle body is visually identifiable.

11. The puncture needle for a photoacoustic imager according to claim 10, wherein
the marking portion is provided in the form of a line extending along the extensional direction of the puncture needle body.

12. The puncture needle for a photoacoustic imager according to claim 10, wherein
the marking portion is provided in the form of a graduation.

13. The puncture needle for a photoacoustic imager according to any of claims 1 to 12, wherein
the semiconductor light-emitting element light source portion includes a light-emitting diode element as a semiconductor light-emitting element (11b).

14. The puncture needle for a photoacoustic imager according to any of claims 1 to 12, wherein
the semiconductor light-emitting element light source portion includes a semiconductor laser element as a semiconductor light-emitting element.

15. The puncture needle for a photoacoustic imager according to any of claims 1 to 12, wherein
the semiconductor light-emitting element light source portion includes an organic light-emitting diode element as a semiconductor light-emitting element.
